(19) Europäisches Patentamt
European Patent Office
Office européen des brevets

(11) **EP 4 576 111 A1**

(12) **EUROPEAN PATENT APPLICATION**

(43) Date of publication:
**25.06.2025 Bulletin 2025/26**

(21) Application number: **24218485.1**

(22) Date of filing: **09.12.2024**

(51) International Patent Classification (IPC):
**G16H 20/30** (2018.01)    **A61B 5/00** (2006.01)
**G16H 30/40** (2018.01)    **G16H 40/63** (2018.01)
**G16H 50/20** (2018.01)    **G16H 50/30** (2018.01)

(52) Cooperative Patent Classification (CPC):
**G16H 20/30; A61B 5/00; G16H 30/40; G16H 40/63; G16H 50/20; G16H 50/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.12.2023  IT 202300027027**

(71) Applicant: **Technogym S.p.A.**
**47521 Cesena (FC) (IT)**

(72) Inventor: **ZOFFOLI, Luca**
**47521 Cesena (FORLI'-CESENA) (IT)**

(74) Representative: **Mozzi, Matteo**
**Jacobacci & Partners S.p.A.**
**Via Senato, 8**
**20121 Milano (IT)**

(54) **METHOD AND SYSTEM FOR DETERMINING VALUES OF METABOLIC PARAMETERS OF A USER WHEN PERFORMING PHYSICAL ACTIVITY ON AN EXERCISE MACHINE**

(57)    A method (600) for determining values of metabolic parameters of a user when performing physical activity on an exercise machine, comprises steps of: acquiring (601), by a thermographic camera, a thermographic image (I-T) of at least one portion of the body of the user when performing physical activity, said at least one portion of the body of the user (U) comprising a body surface of the user; identifying (602), by at least one data processing unit operatively connected to said thermographic camera, within the at least one thermographic image (I-T) acquired by the thermographic camera, a first thermographic portion (P-T1) of the body surface of the user (U); for said identified first thermographic portion (P-T1), determining (603), by the at least one data processing unit, at least one group of thermal features representative of the identified first thermographic portion (P-T1); estimating (604), by the at least one data processing unit, by executing a respective estimation algorithm based on a trained regression model, one or more values of metabolic parameters of the user when performing physical activity on the exercise machine based on said at least one determined group of thermal features representative of said identified first thermographic portion (P-T1), said one or more values of metabolic parameters of the user (U) comprising at least one of the following: value of oxygen volume ($VO_2$) that the user is consuming when performing physical activity; value of carbon dioxide volume ($VCO_2$) that the user is producing when performing physical activity by the user; value of respiratory quotient (RQ) of the user when performing physical activity by the user; providing (605), by the at least one data processing unit, said estimated one or more values of metabolic parameters.

Fig. 6

**Description**

Field of the invention

The present invention relates to the fitness field, and in particular to a method and related system for determining values of metabolic parameters of a user when performing physical activity on an exercise machine.

Technological background of the invention

When performing physical activity by a user, in particular aerobic training, knowledge of the current metabolic state of the user is highly important to define and adapt a physical exercise in accordance with the actual capabilities of the body of the user.

However, obtaining information representative of the metabolic state of the body is not trivial and often requires the availability of rather expensive instruments such as, for example, so-called gas exchange analyzers.

Alternatively, less expensive sensors are generally used (for example, heart rate monitoring sensors) which, however, are instruments with a very limited effectiveness in this respect, since they can only provide an indirect measurement of the actual metabolic state.

Therefore, nowadays, the need is strongly felt to have alternative techniques available for determining values of metabolic parameters of a user when performing physical activity on an exercise machine which allow obtaining information representative of metabolic and physiological parameters of the body of a user during aerobic training which is as precise and reliable as possible.

Summary

It is the object of the present invention to devise and provide a method for determining values of metabolic parameters of a user when performing physical activity on an exercise machine which allows obviating at least partially the drawbacks complained of above with reference to the prior art, in particular which allows obtaining information representative of metabolic and physiological parameters of the body of a user during aerobic training which is as precise and reliable as possible.

Such an object is achieved by a method in accordance with claim 1.

The present invention also relates to a system for determining (estimating) values of metabolic parameters of a user when performing physical activity on an exercise machine.

Preferred embodiments of said method and said system are defined in the respective dependent claims.

The present invention also relates to a method for controlling an exercise machine when performing physical activity by the user, which utilizes the metabolic parameters determined with the method and system in accordance with the present invention.

Brief description of the Figures

Further features and advantages of the method and the related system according to the invention will become apparent from the following description of preferred embodiments, given by way non-limiting indication, with reference to the accompanying drawings, in which:

- Figure 1 diagrammatically shows a system for determining values of metabolic parameters of a user when performing physical activity on an exercise machine, in accordance with an embodiment of the present invention;
- Figure 2 diagrammatically shows a system for determining values of metabolic parameters of a user when performing physical activity on an exercise machine, in accordance with a further embodiment of the present invention;
- Figure 3a diagrammatically shows a first example of a thermographic image acquirable and usable when performing the method for determining values of metabolic parameters of a user when performing physical activity on an exercise machine, in accordance with the present invention;
- Figure 3b shows a second example of a thermographic image acquirable and usable when performing the method for determining values of metabolic parameters of a user when performing physical activity on an exercise machine, in accordance with the present invention;
- Figure 3c shows a subsequent processing of the thermographic image in Figure 3b;
- Figure 3d shows a further subsequent processing of the thermographic image in Figure 3b as a function of the subsequent processing in Figure 3c;
- Figure 4 diagrammatically shows an example of a neural network usable during when performing the method for determining values of metabolic parameters of a user when performing physical activity on an exercise machine, in accordance with the present invention;

- Figure 5 diagrammatically shows, by means of a flow diagram, an example of a method for determining values of metabolic parameters of a user when performing physical activity on an exercise machine;
- Figure 6 diagrammatically shows, by means of a block diagram, a method for determining values of metabolic parameters of a user when performing physical activity on an exercise machine, in accordance with the present invention, and
- Figure 7 diagrammatically shows, by means of a block diagram, a method for controlling an exercise machine when performing physical activity by the user, which uses the metabolic parameters determined with the method and the system in accordance with the present invention.

It should be noted that, in the aforesaid Figures, equivalent or similar elements are indicated by the same numeric and/or alphanumeric reference.

Detailed description

With reference to the above Figures, a system 100 is now described for determining (estimating or predicting) values of metabolic parameters of a user when performing physical activity on an exercise machine, in accordance with the present invention.

The system 100 comprises at least one exercise machine 1 on which a user U can perform physical activity.

"Exercise machine" means any exercise machine suitable for cardiovascular activity such as, for example, a treadmill, a bike or an exercise bike, a rower and so on, any exercise machine suitable for muscle strengthening activity such as, for example, a leg press, a chest press and so on, or an exercise machine suitable for performing physical mobility or postural exercises or the like.

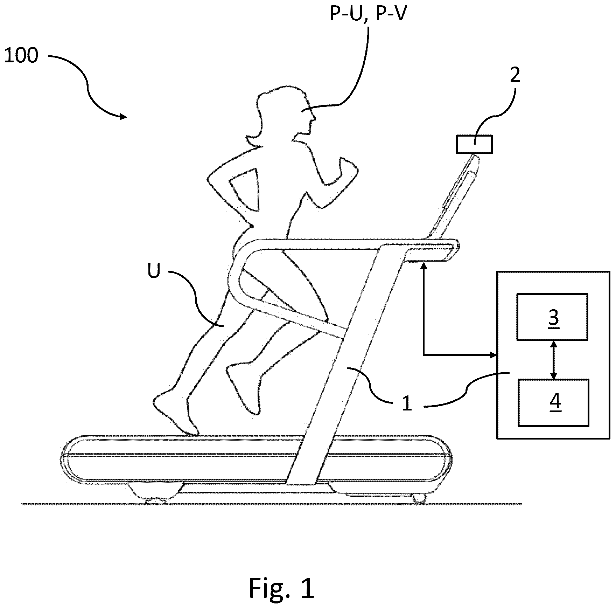

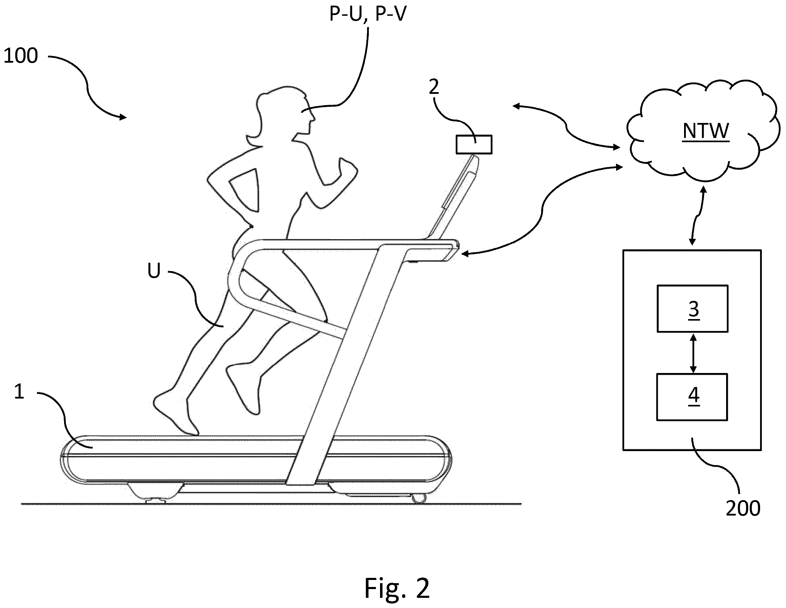

The example of exercise machine 1 shown in Figures 1 and 2 is a treadmill.

The system 100 further comprises a thermographic camera 2.

For the purposes of the present invention, "thermographic camera" means any camera sensitive to infrared radiation of the electromagnetic spectrum capable of detecting a set range of infrared values of the electromagnetic spectrum, having in particular wavelengths preferably from 7 to 14 micrometers ($\mu$m).

The thermographic camera 2 is configured to acquire at least one thermographic image I-T of at least one portion P-U of the body of the user U when performing physical activity on the exercise machine 1.

The at least one portion P-U of the body of the user comprises a body surface P-V of the user U.

According to an embodiment, in combination with any of the preceding and subsequent ones, the body surface P-V of the user U comprised in the at least one portion P-U of the body of the user of the at least one acquired thermographic image I-T is free of clothing, for example the face and neck of the user.

"Thermographic image" means a graphic representation of the intensity of infrared radiation emitted by the space displayed (for example, the body surface in the case of a user).

In greater detail, such a graphic representation corresponds to a matrix in which each element provides a value representative of the temperature detected in the thermographic image.

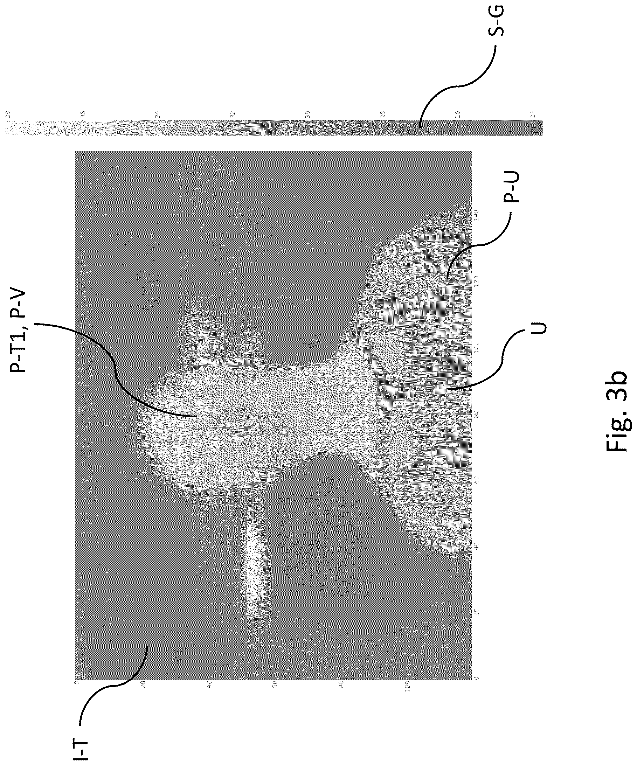

As shown in the example in Figure 3b, the thermographic image is constructed by associating a predefined color scale to the individual temperature values detected, such as to allow the creation of an image based on the temperature value detected for each pixel of the thermographic image.

Conventionally, the color scale ranges from warm colors, which correspond to the highest temperature values, to cold colors, which correspond to the lowest temperature values.

In this respect, Figure 3b, which will also be referred to below, shows an example of a thermographic image (albeit in shades of gray) with a vertically adjacent gray scale S-G.

Again with reference, in general, to the present invention, a control unit on board the thermographic camera 2 is configured to generate, from the detected infrared radiation, temperature maps of the exposed surface of the item observed.

In greater detail, the control unit on board the thermographic camera 2 is configured to quickly acquire the value of energy stored by each single pixel, transform it into a temperature value and generate a thermographic image of the item observed based on a predefined color scale.

In one embodiment, the thermographic camera 2 comprises a thermal camera.

In a further embodiment, alternative to the preceding one, the thermographic camera 2 comprises an infrared camera.

In other words, in one embodiment, the thermographic camera 2 comprises a thermal camera or an infrared camera.

In one embodiment, shown in Figures 1 and 2, the thermographic camera 2 is installed on or integrated into the exercise machine 1 and is oriented so as to acquire the thermographic images of said at least one portion P-U of the body of the user U when performing physical activity on the exercise machine 1.

In accordance with a further embodiment, alternative to the preceding one and not shown in the Figures, the thermographic camera 2 is separated from the exercise machine 1 and is installed inside the training environment (for example, on a wall of a gym) in which the exercise machine 1 is located and usable by the user 1 to perform the physical activity.

In accordance with a further embodiment, alternative to the preceding ones and not shown in the Figures, the thermographic camera 2 is separated from the exercise machine 1 since it is integrated (connected integrally or in a removable manner) in a portable electronic device of the user (for example, a smartphone or a tablet) present within the training environment in which the exercise machine 1 is located and usable by the user U to perform physical activity. The portable electronic device of the user is, in this case, configured, for example by loading and executing a related software application (APP), to receive and process the data detected by the thermographic camera integrated therein.

Also in the aforesaid embodiments, the thermographic camera 2 is positioned and oriented so as to acquire the thermographic images of said at least one portion P-U of the body of the user U when performing physical activity on the exercise machine 1.

Again with reference, in general, to the present invention and to Figures 1 and 2, the system 100 further comprises at least one data processing unit 3, for example a microcontroller or a microprocessor, operatively connected to the thermographic camera 2.

The system 100 further comprises at least one memory unit 4 operatively connected to the at least one data processing unit 3.

The at least one data processing unit 3 is configured to load and execute one or more program codes (previously stored in the at least one memory unit 4) to perform steps of the method for determining (estimating) metabolic parameters of a user when performing physical activity on an exercise machine of the present invention as described below.

In one embodiment, shown in Figure 1, the at least one data processing unit 3 and the at least one memory unit 4 are integrated into the exercise machine 1.

In this respect, it should be noted that in Figure 1 the at least one data processing unit 3 and the at least one memory unit 4 are diagrammatically shown outside the exercise machine 1, exclusively for ease of representation.

In greater detail, the at least one data processing unit 3 and the at least one memory unit 4 can be in addition to or coincide with a data processing module and the respective memory module (not shown in the Figures) already present in the exercise machine 1 and configured to allow the exercise machine 1 to operate correctly and be used by the user U to perform physical activity.

Therefore, in this embodiment, the data processing part provided by the method for determining (estimating) metabolic parameters of a user when performing physical activity on an exercise machine of the present invention is executable locally by the exercise machine 1.

In accordance with a further embodiment, shown in Figure 2, the at least one data processing unit 3 and the at least one memory unit 4 reside in a remote electronic processor 200 operatively connected to the thermographic camera 2 and the exercise machine 1.

In greater detail, the connection between the at least one data processing unit 3 and the thermographic camera 2 and the exercise machine 1 occurs in wireless mode or in wired mode, by means of a data communication network NTW, diagrammatically shown in Figure 2, for example Internet.

Therefore, in this embodiment, the data processing part provided by the method for determining (estimating) metabolic parameters of a user when performing physical activity on an exercise machine of the present invention is executable by the remote electronic processor 200.

According to a further embodiment, as an alternative to the two preceding ones, the at least one data processing unit 3 and the at least one memory unit 4 can be partly integrated into the exercise machine 1 and partly integrated into the remote electronic processor 200.

Therefore, in this embodiment, the data processing part provided by the method for determining (estimating) metabolic parameters of a user when performing physical activity on an exercise machine of the present invention is executable partly by the exercise machine 1 and partly by the remote electronic processor 200.

According to a further embodiment, in combination with the preceding one, the system 100 can comprise one or more data processing units 3, and one or more respective memory units 4, so as to perform the respective functions, depending on the configuration, totally locally, i.e., by the exercise machine 1, totally remotely with respect to the training environment in which the exercise machine 1 is located, i.e., by the remote electronic processor 200, or in a hybrid mode, i.e., with functions executable locally by the exercise machine 1 and functions executable remotely by the remote electronic processor 200.

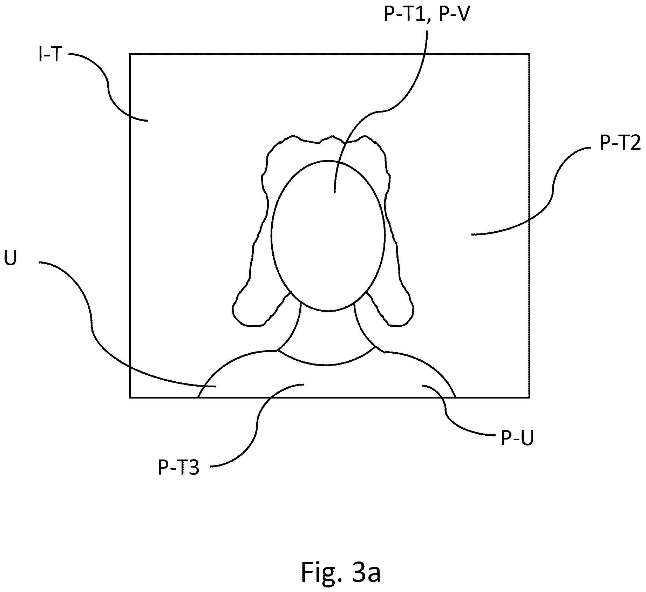

Turning back, in general, to the present invention and referring now to Figures 3a and 3b, the at least one data processing unit 3 operatively connected to said thermographic camera 2, is configured to identify within a thermographic image I-T acquired by the thermographic camera 2 a first thermographic portion P-T1 representative of the body surface P-V of the user U without clothing (for example, the face and neck of the user U).

It should be noted that the first thermographic portion P-T1 representative of the body surface P-V of the user U which the at least one data processing unit 3 is capable of identifying within the thermographic image I-T acquired by the thermographic camera 2 is also defined as the region of interest or simply ROI.

The identification of such a portion is obtained by the at least one data processing unit 3, for example, by implementing a segmentation technique of the acquired thermographic image I-T.

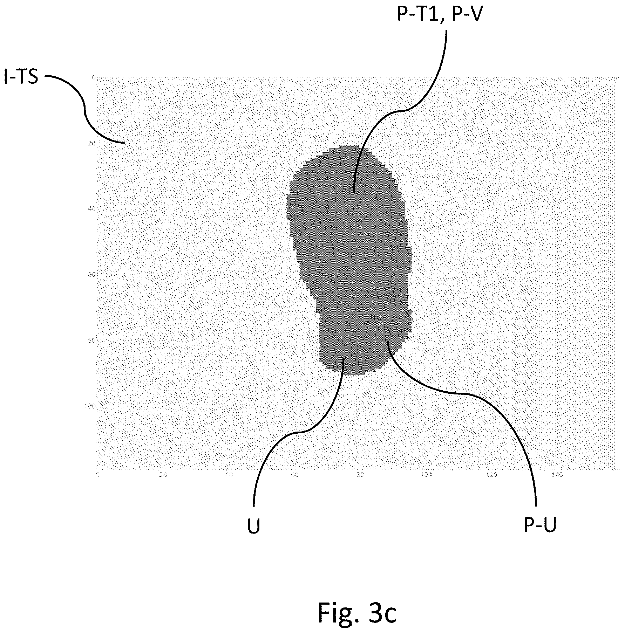

An example of the result of the implementation of a segmentation technique, applied for example to the thermographic image in Figure 3b, is the segmented thermographic image I-TS shown in Figure 3c.

As shown in Figure 3c, following segmentation, in the segmented thermographic image I-TS the first region of interest corresponding to the face and neck of the user is identifiable.

The segmented thermographic image I-TS corresponds to a matrix which assigns different numeric values (defined as arbitrary "weights") to the regions obtained from the segmentation.

In the example in Figure 3c, the matrix corresponding to the segmented thermographic image I-TS assigns a first value (for example, the value one) to each pixel of the first region of interest and a second value (for example, the value zero) to each pixel not belonging to the first region of interest.

In other words, from a numerical point of view, in the segmented thermographic image I-TS, all the pixels of the first thermographic portion P-T1 representing the body surface P-V of the user U (region of interest) have assigned the same first value (for example, one) while all the pixels of the remaining portion (region of non-interest) have assigned the same second value (for example, zero).

From a graphical point of view, as shown in the example in Figure 3c, all the pixels of the segmented thermographic image I-TS to which the first value has been assigned are represented with the same first color (in the example in Figure 3c, dark grey) while all the pixels of the segmented thermographic image I-TS to which the second value has been assigned are represented with the same second color (in the example in Figure 3c, light grey), different and distinguishable from the first color.

In other words, from a graphical point of view, in the segmented thermographic image I-TS, the first thermographic portion P-T1 representative of the body surface P-V of the user U (region of interest) is of a first color (dark gray in the example in Figure 3c) while the remaining portion (region of non-interest) is of a second color (light gray in the example in Figure 3c).

Moreover, it should be noted that the matrix corresponding to the segmented thermographic image I-TS represents a filter function applicable to the at least one acquired thermographic image I-T by multiplying the matrix corresponding to the at least one acquired thermographic image I-T (starting matrix) by the matrix corresponding to the segmented thermographic image I-TS.

In fact, for example, if the first value is one and the second value is zero, by multiplying the matrix corresponding to the at least one acquired thermographic image I-T (starting matrix) by the matrix corresponding to the segmented thermographic image I-TS, a matrix is obtained which comprises the same values of the starting matrix at the elements which have been multiplied by the first value (one) while it comprises the value zero in all the elements which have been multiplied by the second value (zero).

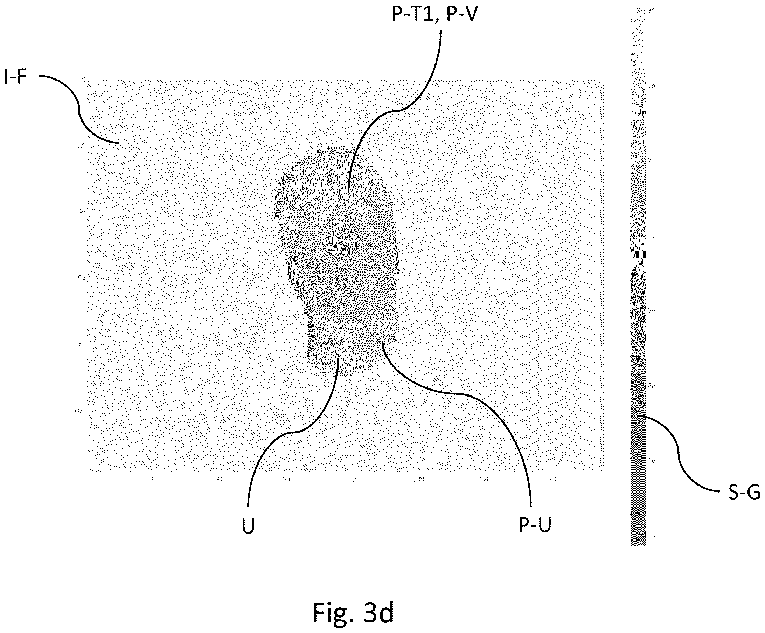

The result of such a processing is shown in the example in Figure 3d, in which a filtered thermographic image I-F is shown comprising as the first thermographic portion P-T1 representative of the body surface P-V of the user U (region of interest) the one shown in shades of gray in Figure 3b (with the corresponding gray scale S-G vertically next to it) and as the remaining portion (region of non-interest) the colored portion in the second color (light gray) as shown in Figure 3c.

Again with reference, in general, to the present invention, the at least one data processing unit 3, for said identified first thermographic portion P-T1, is configured to determine at least one group of thermal features representative of the identified first thermographic portion P-T1.

"At least one group of thermal features" means one or more thermal features representative of the first thermographic portion P-T1 identified by processing digital pixels of said identified first thermographic portion P-T1.

In greater detail, the at least one data processing unit 3, for said identified first thermographic portion P-T1, is configured to determine the at least one group of thermal features representative of the first thermographic portion P-T1 identified by processing digital pixels of said identified first thermographic portion P-T1.

For example, as mentioned above, the matrix corresponding to the at least one acquired thermographic image I-T (Figure 3b) is multiplied by the matrix corresponding to the segmented thermographic image I-TS (Figure 3c), so as to filter the at least one acquired thermographic image I-T and identify the pixels corresponding to the first thermographic portion P-T1 (region of interest) thus identified. It is thus possible to obtain a filtered thermographic image I-F (Figure 3d).

The pixels of the at least one filtered thermographic image I-T corresponding to the identified first thermographic portion P-T1 (region of interest) are then processed so as to provide, in the form of a numeric value, each thermal feature of the at least one group of thermal features representative of the first thermographic portion P-T1.

Therefore, each thermal feature corresponds to a numeric value.

Examples of thermal features, associated with said identified first thermographic portion P-T1, following the processing of a set number of digital pixels of said identified first thermographic portion, are, for example:

- mean temperature value;
- minimum temperature value;
- maximum temperature value;
- standard deviation of temperature;
- interquartile range;
- variation coefficient;
- amplitude ratio;
- entropy;
- contrast;
- homogeneity;
- spatial correlation;
- surface (area or number of pixels).

The at least one data processing unit 3, by executing a respective estimation algorithm based on a trained regression model, is configured to estimate (or predict) one or more values of metabolic parameters of the user U when performing physical activity on the exercise machine 1 based on said at least one determined group of determined thermal features representative of said identified first thermographic portion P-T1.

In fact, for the purposes of the present invention, the physiological assumption is applied according to which the heat dispersion by humans, the latter being homeothermic organisms, provides an indication of the values of the metabolic parameters of humans which vary as a function of the heat dispersion.

Said one or more values of metabolic parameters of the user U comprise at least one of the following:

- value of oxygen volume ($VO_2$) that the user U is consuming when performing physical activity;
- value of carbon dioxide volume ($VCO_2$) that the user U is producing when performing physical activity by the user;
- value of respiratory quotient or RQ of the user U when performing physical activity by the user.

According to an embodiment, the at least one data processing unit 3, by executing a respective estimation algorithm based on a trained regression model, is configured to estimate (or predict) a first value of a first metabolic parameter and a second value of a second metabolic parameter of said one or more values of metabolic parameters.

In this embodiment, the at least one data processing unit 3 is configured to determine a third value of a third metabolic parameter of said one or more values of metabolic parameters based on the estimated first value of the first metabolic parameter and the estimated second value of the second metabolic parameter of said one or more values of metabolic parameters.

In fact, the value of respiratory quotient or RQ is the ratio between the value of carbon dioxide volume ($VCO_2$) that the user U is producing when performing physical activity and the value of oxygen volume ($VO_2$) that the user U is consuming when performing physical activity.

The value of respiratory quotient RQ provides a representative indication of the intensity of the physical exercise and also allows determining the energy substrates, i.e., how much the user U, during the physical activity, is consuming in terms of fats and sugars.

Conventional values of the respiratory quotient RQ are 0.6 - 0.8 at rest and 1.2 - 1.3 under maximal effort.

Therefore, when considering the relationship $RQ = VCO_2 / VO_2$, the data processing unit 3, once estimated two values of metabolic parameters between RQ, VCO2 and VO2, is configured to determine the other (not estimated) value of metabolic parameter as a value deriving from the two estimated values of metabolic parameters, using the aforesaid relationship.

In the light of the above, the following cases are envisaged:

a) estimate of values of $VCO_2$ and $VO_2$ -> derived value of $RQ = VCO_2 / VO_2$;
b) estimation of values of RQ and $VO_2$ -> derived value of $VCO_2 = RQ \times VO_2$;
c) estimation of values of RQ and $VCO_2$ -> derived value of $VO_2 = VCO_2 / RQ$.

Therefore, the fact of estimating two values of metabolic parameters with the trained regression model and a third value of metabolic parameter only by derivation from the other two estimated values of metabolic parameters represents an apparent advantage also from a computational point of view.

According to an embodiment, in combination with any of the preceding ones, said one or more values of metabolic parameters of the user U further comprise values deriving from one or more of said one or more values of metabolic parameters.

For example, such values deriving from one or more of said one or more values of metabolic parameters comprise:

- value of caloric consumption (kcal/h), correlated to the value of oxygen volume ($VO_2$) that the user is consuming when performing physical activity;

- value of quantity of calories deriving from the consumption of fats and sugars, correlated to the value of respiratory quotient (RQ) of the user U when performing physical activity.

With reference to the caloric consumption (kcal/h), see for example the mathematical relations:

$$VO_2/3.5 = MET,$$

where MET is the Metabolic Equivalent of Task

$$MET \times (user\ weight) = kcal/h$$

According to an embodiment, the estimation algorithm based on a trained regression model is based on the use of a set regression technique for estimating (or predicting) said one or more values of metabolic parameters of the user U when performing physical activity on the exercise machine 1.

The at least one data processing unit 3 obtains the estimated (or predicted) value of a metabolic parameter of the user U at the instant of time of acquisition of the at least one thermographic image I-T by solving equations of a trained regression model, inserting as input the values of said at least one determined group of thermal features representative of said identified first thermographic portion P-T1.

An example of a regression technique can be the multiple regression technique the trained model of which is representable by the following relation (1):

$$y_h = \sum_{k=1}^{s} \sum_{j=0}^{m_k} \beta_{kj} \cdot x_{kh}^{j} + \epsilon \qquad (1)$$

where:

- $y_h$ is the value of the metabolic parameter to be estimated, in an observation h, with $1 < h < n$ (n set number of observations) (i.e., in an instantaneous acquisition of a thermographic image);

- $x_{kh}$, with $1 < k < s$, are the values of the (known) thermal features representative of said identified first thermographic portion P-T1, i.e., the values of the prediction variables, in the observation h (i.e., in the instantaneous acquisition of a thermographic image);

- $\beta_{kj}$ are the values of coefficients (weights) of the model associated with each thermal feature $x_k$ and degree j of the polynomial;

- $m_k$ is the degree of the polynomial for the value of the thermal feature Xk,

- $\varepsilon$ is the error value of the estimate.

The linear regression can be simple or multiple.

In the case of a simple linear regression technique, the trained model is represented by the equation of a straight line which models the relationship of the value $y_h$ to be estimated with a single prediction variable $x_{hk}$.

Therefore, in accordance with the present invention, the linear regression line models the relationship of a value of metabolic parameter to be estimated with a determined thermal feature representative of said identified first thermographic portion.

An example of an equation (model) provided by the simple linear regression line is one in which there is only the prediction variable $x_h$ (s=1) with degree $m_k$=1.

The multiple linear regression technique is a generalized case (already contemplated in the mathematical relationship (1) reported above) of the simple linear regression technique in which multiple values $x_1, ... x_s$ of thermal features are considered to predict the respective dependent variable $y_h$, where h, with $1 < h < n$ (n set number of observations).

Therefore, in accordance with the present invention, the linear regression model models the relationship, for a set number of observations (i.e., instantaneous acquisitions of thermographic images), of a value of metabolic parameter to be estimated with at least one thermal feature of said at least one group, determined by the data processing unit 3, of thermal features representative of the first thermographic portion P-T1 in turn identified, by the data processing unit 3, within the thermographic image I-T acquired by the thermographic camera.

As for the training of the regression model to be used in the case of linear regression technique, an example of training consists in the application of the least squares regression technique, which is based on a set number of acquisitions or observations.

According to an embodiment, the estimation algorithm, based on a regression model, is based on the application of a neural network (therefore, through the execution of artificial intelligence techniques) for estimating (or predicting) said one or more metabolic parameters of the user U when performing physical activity on the exercise machine 1.

In particular, by definition, the neural network is an example of a regression model used to classify said one or more values of metabolic parameters.

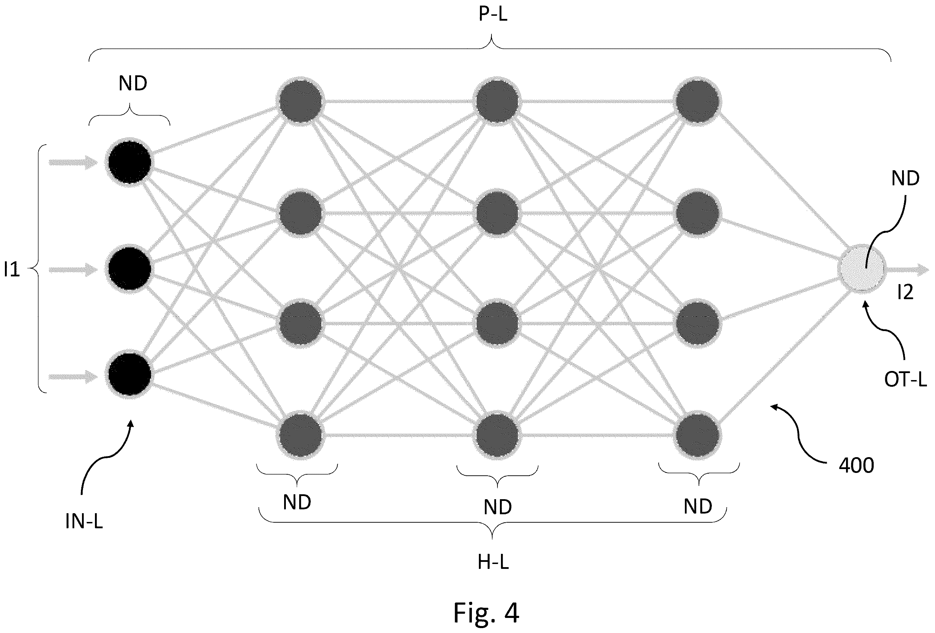

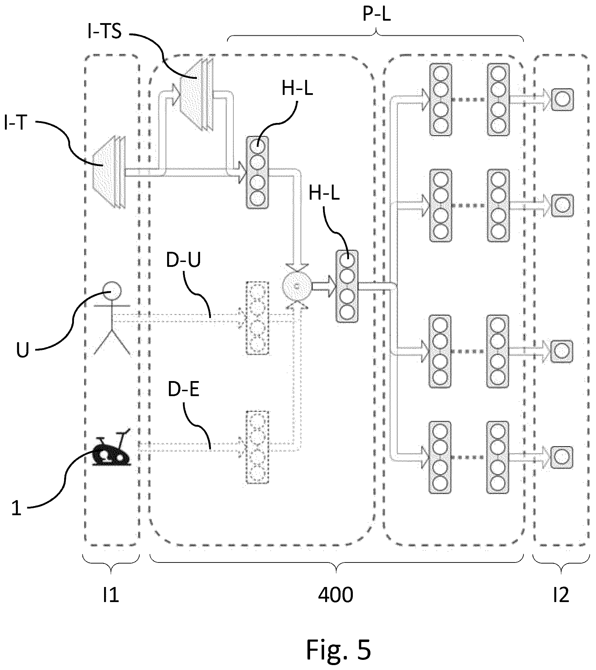

The neural network, indicated with reference numeral 400 in Figure 4 and Figure 5, is for example a neural network of the convolutional type (Convolutional Neural Network, CNN).

A neural network 400 of the convolutional type CNN comprises a plurality of layers P-L, i.e., an input layer IN-L, an output layer OT-L and a plurality of intermediate layers H-L.

Each layer comprises a plurality of nodes ND interconnected with one another.

The nodes of different layers are interconnected with one another.

The input data I1 of a neural network are subject to the application of set decision rules (weights) which are present in each connection between the interconnected nodes, from the input layer N-L up to the output layer OT-L as output data I2, passing through the plurality of intermediate layers H-L (also called hidden layers). In this respect, the nodes of the intermediate layers are also called hidden nodes.

It should be noted that the greater the number of nodes, and therefore the number of connections between interconnected nodes, through which an input data I1 can pass, the greater the precision of the information which will be contained in the output data I2.

By way of example, if the input data I1 are the values of said at least one determined group of thermal features representative of said identified thermographic portion P-T1, the output data I2 processed by the neural network 400 will be the estimated values of said one or more values of metabolic parameters of the user U when performing physical activity on an exercise machine 1.

The weight of each connection between interconnected nodes ND of the neural network 400 is determined by training the neural network.

In this respect, the training of the neural network involves the following.

The user U trains on the exercise machine 1 (for example, a treadmill) while keeping the exercise parameters constant (for example, the speed value and slope value of the treadmill).

The activity is maintained until a condition of metabolic stability or stationary state of the user is reached for a set first time interval, for example one minute.

It should be noted that the set first time interval is between 30 seconds and a few minutes (at least 3 or more).

In particular, "stationary state of the user" means a condition for which the monitored metabolic parameters remain within a pre-set "stable interval" for the set first time interval.

For example, when detecting the value of the heart rate of the user, having defined as a "stable condition" a variation of less than 3% (negative or positive) with respect to a theoretical maximum value $FC_{max}$ calculated for the user, reaching the "stationary state of the user" will correspond to the moment in which such a "stable condition" is maintained for a set time interval (for example, one minute).

The theoretical maximum value $FC_{max}$ calculated for the user used in this example can be calculated by using, for example, the following relationship: $FC_{max} = 207 - 0.7 \times$ (age of the user).

After the set first time interval (for example, one minute), in a second set time interval (for example, one minute), the values of metabolic parameters of interest are acquired by means of the use of specific sensors such as, for example: a monitor for detecting a value of the heart rate and/or a gas exchange analyzer, capable of measuring a value of oxygen volume $VO_2$ that the user is consuming by means of the gas exchanges which occur while breathing.

It should be also noted that the set second time interval is between 30 seconds and a few minutes (at least 3 or more).

In any case, the set second time interval can have the same duration as the set first time interval or another duration (longer or shorter).

The acquired values of metabolic parameters are stored in an additional memory unit of an electronic computer or in an electronic apparatus dedicated to this step of training of the neural network.

The acquisition ends after the set second time interval, for example one minute.

At the end of the set second time interval, possible objects/sensors covering/hiding the body/face or portions thereof are removed without interrupting the physical activity of the user U and, at the same time, the acquisition begins, by the thermographic camera 2, of thermographic images I-T of at least one portion P-U of the body of the user U when performing physical activity on the exercise machine 1 in conditions comparable to those of normal use by the user.

The acquired thermographic images I-T are stored in the further memory unit of the electronic computer or of the electronic apparatus dedicated to this step of training of the neural network.

At the end of the acquisition, the acquired values of metabolic parameters of interest are appropriately processed (for example, by applying filters and/or control logics depending on the metabolic parameter analyzed) by a further data processing unit (for example, an electronic calculator or electronic apparatus dedicated to this step of training of the neural network) so as to remove any disturbances or artefacts in the acquired signal, and the mean values and standard deviation values thereof are calculated.

Also at the end of the acquisition, the acquired thermographic images are processed by the further data processing unit (for example, of the electronic calculator or electronic apparatus dedicated to this step of training of the neural network) as indicated above (i.e., segmentation, feature extraction, and so on).

Starting from the assumption that the stationary state was maintained throughout the entire duration of the set second time interval in which the values of metabolic parameters of interest were acquired, for each frame captured by the thermographic camera 2, the further data processing unit (for example, of the electronic calculator or the electronic apparatus dedicated to this training step of the neural network) defines, for each metabolic parameter of interest, a random value defined within a Gaussian distribution having a mean and standard deviation equal to that calculated as described above.

This approach has the function of associating the thermal features extracted from each thermographic image with a range of values of metabolic parameters consistent with what was collected experimentally (a technique known as Data Augmentation).

Again with reference to the neural network, it is therefore trained (i.e., calculating the values of the weights to be associated with each connection between the interconnected nodes of the intermediate or hidden layers of the neural network 400) using as input data I1 to the neural network 400 thermal input data as well as, optionally, personal and/or anthropometric data of the user (e.g.: age, gender, weight, height, BMI, and so on) and as output data OT-L of the neural network 400 the metabolic parameters determined as above.

In greater detail, with reference to the regression model represented by the neural network, the above description allows building a dataset with which to train the neural network, i.e., it allows having values of the prediction variable x (thermal features associated with thermographic portions of the acquired thermographic images) and values of the variable y to be estimated (values of metabolic parameters) of the model.

The training of the neural network consists in determining the values of coefficients, i.e., the values of the weights to be associated with each connection between the interconnected nodes of the intermediate or hidden layers of the neural network 400, such as to minimize the estimation error of the metabolic parameters from the input thermal features.

In this respect, an example of a training technique is the training technique called "Back Propagation".

Again with reference, in general, to the estimation algorithm based on the trained regression model, it should be noted that such a trained regression model, in an embodiment, can be loaded into the data processing unit 3 to improve, from a computational point of view, the data processing and the response speed by the data processing unit 3.

The system 100 of the invention and the related method, described below, are advantageously usable in real-time applications.

Turning back now, in general, to the invention, the at least one data processing unit 3 is configured to provide said one or more values of estimated (or predicted) metabolic parameters.

In greater detail, the values of estimated (or predicted) metabolic parameters can be provided by the at least one data processing unit 3 to a portable electronic device of the user U, to an electronic device or an electronic apparatus of a personal trainer, to the remote electronic computer 200 (if present).

In this respect, it should be noted that the values of estimated metabolic parameters can in turn be used to estimate a value of caloric expenditure (calories burned) of the user U when performing physical activity.

For example, given the value of oxygen volume $VO_2$ that the user U is consuming at each instant of time of acquisition of the thermographic images I-T, the time interval when performing the physical activity and the weight of the user U, it is possible to determine the value of the caloric expenditure of the user U that the user U is consuming during the time interval when performing the physical activity on the exercise machine 1, for example, using the mathematical relationships already indicated above.

Moreover, the value of respiratory quotient or RQ of the user U when performing physical activity (for example, at each instant of time of acquisition of thermographic images I-T) and the value of oxygen volume $VO_2$ that the user U is consuming when performing physical activity (for example, at each instant of time of acquisition of thermographic images I-T), can be used to determine, by the at least one data processing unit 3 or the remote electronic processor 200, if present, the maximum volume $VO_{2max}$ of oxygen that the user U can consume in a time interval when performing physical activity on the exercise machine 1.

For example, the determination of such a value can be obtained by taking into account the following mathematical relationship which correlates the value of respiratory quotient RQ to the percentage of oxygen volume consumed by the user with respect to the maximum oxygen volume $VO_{2max}$ that the user can consume when performing physical activity on the exercise machine 1:

$$RQ = c_1 \times (\%VO_{2max})^2 + c_2 \times \%VO_{2max} + c_3 \qquad (3)$$

where $_1$, $c_2$ and $c_3$ are coefficients determined/estimated a priori.

According to an embodiment, in combination with any of the preceding ones, the at least one data processing unit 3, by executing the estimation algorithm based on a trained regression model, is configured to estimate (or predict) said one or more values of metabolic parameters of the user U when performing physical activity on the exercise machine 1 based on, in addition to the determined at least one group of determined thermal features representative of said identified first thermographic portion P-T1, first data D-E representative of the physical exercise being performed by the user U provided by the exercise machine 1 and/or second anthropometric data D-U of the user U (diagrammatically indicated in Figure 5).

It is thus possible to obtain a better estimate.

"First data representative of the physical exercise being performed by the user U provided by the exercise machine 1" means data of the exercise machine, dependent on the type of exercise machine used by user U.

For example, such first data can be:

- running speed and inclination of the treadmill;
- power and pace of a bike;
- load lifted and speed of movement and/or power for a strength machine or machine adapted to perform mobility or postural physical exercises.

"Anthropometric data of the user U" means personal data such as age, weight, height, gender, and so on.

The second anthropometric data D-U of the user U can be provided manually or reside in, and therefore be retrievable from, a remote database, so as to be available following an authentication procedure of the user U.

Moreover, the second anthropometric data D-U of the user U, as an alternative to being obtained following the authentication step of the user U, can be generated/updated from time to time by the at least one data processing unit 3 when using the exercise machine 1.

In accordance with an embodiment, in combination with any of the preceding ones, the at least one data processing unit 3 is configured to store within the at least one memory unit 4 operatively connected to said at least one data processing unit 3, said determined groups of thermal features representative of said identified first thermographic portion P-T1.

In accordance with an embodiment, in combination with any of the preceding ones, the at least one data processing unit 3 is configured to store inside the at least one memory unit 4 operatively connected to said at least one data processing unit 3, said estimated one or more values of metabolic parameters associated with the acquired at least one thermographic image 1.

In an embodiment, in combination with any of the preceding ones, the at least one data processing unit 3 is configured to perform the operations described above in an instant of time of acquisition of a single thermographic image I-T acquired by the thermographic camera 2 and based on the processing of said single thermographic image I-T acquired by the thermographic camera 2.

In this embodiment, said one or more values of metabolic parameters which can be estimated comprise:

- value of oxygen volume $VO_2$ that the user U is producing at the instant of time of acquisition of the at least one thermographic image I-T;
- value of carbon dioxide volume $VCO_2$ that the user U is producing at the instant of time of acquisition of the at least one thermographic image I-T;
- value of respiratory quotient RQ of the user U in the instant of time of acquisition of at least one thermographic image I-T.

According to an embodiment, the at least one data processing unit 3, by executing a respective estimation algorithm based on a trained regression model, is configured to estimate (or predict) a first value of a first metabolic parameter and a second value of a second metabolic parameter of said one or more values of metabolic parameters.

In this embodiment, the at least one data processing unit 3 is configured to determine a third value of a third metabolic parameter of said one or more values of metabolic parameters based on the estimated first value of the first metabolic parameter and the estimated second value of the second metabolic parameter of said one or more values of metabolic parameters.

In fact, the value of respiratory quotient or RQ is the ratio between the value of carbon dioxide volume ($VCO_2$) that the user U is producing at the instant of time of acquisition of the at least one thermographic image I-T and the value of oxygen volume ($VO_2$) that the user U is consuming at the instant of time of acquisition of the at least one thermographic image I-T.

Therefore, when considering the relationship $RQ = VCO_2 / VO_2$ (already indicated above), the data processing unit 3, once estimated two values of metabolic parameters between RQ, $VCO_2$ and $VO_2$, is configured to estimate the other (not estimated) value of metabolic parameter as a value deriving from the two estimated values of metabolic parameters, using the aforesaid relationship (cases of this type have already been indicated above).

Therefore, also in this embodiment, the fact of estimating two values of metabolic parameters with the trained regression model and a third value of metabolic parameter only by derivation from the other two estimated values of metabolic parameters represents an apparent advantage also from a computational point of view.

According to an embodiment, in combination with any of the preceding ones, wherein the previous steps are performed by the at least one data processing unit 3 in an instant of time of acquisition of a single thermographic image I-T acquired by the thermographic camera 2 and based on the processing of said single thermographic image I-T acquired by the thermographic camera 2, said one or more values of metabolic parameters of the user U further comprise values deriving from one or more of said one or more values of metabolic parameters.

For example, such values deriving from one or more of said one or more values of metabolic parameters comprise:

- value of caloric consumption (kcal/h), correlated to the value of oxygen volume ($VO_2$) that the user produces at the instant of time of acquisition of the at least one thermographic image I-T;
- value of the quantity of calories deriving from the consumption of fats and sugars, correlated to the value of respiratory quotient (RQ) of the user U at the instant of time of acquisition of the at least one thermographic image I-T.

For example, in the case of estimating the value of oxygen volume that the user U is consuming at the instant of time of acquisition of the at least one thermographic image I-T, such a value of metabolic parameter can be estimated, by means of the estimation algorithm based on the trained regression model, from a vector of thermal features (i.e., a single-dimensional matrix), therefore without taking into account the time variable.

In accordance with a further embodiment, in combination with any of the preceding ones, the at least one data processing unit 3 is configured to repeat the operations described above for a plurality of instants of time of acquisition included in a time interval and based on the processing of a plurality of thermographic images I-T acquired by the thermographic camera 2 in said time interval.

A thermographic image I-T is acquired in an instant of time of acquisition of said plurality of instants of time of acquisition.

In this embodiment, in addition to the values of metabolic parameters already listed above, the estimate of which would thus be more reliable, said one or more values of metabolic parameters of the user U further comprise:

- value of heart rate of the user U when performing physical activity on the exercise machine 1;
- value of respiratory rate of the user U when performing physical activity on the exercise machine 1.

The value of the heart rate or the value of the respiratory rate are in fact correlated to the variation of the value of oxygen volume ($VO_2$) that the user U consumes in a time interval when performing physical activity on the exercise machine 1.

For example, in the case of the estimate of the value of the heart rate of the user in the time interval when performing physical activity by the user on the exercise machine 1, such a value of metabolic parameter can be estimated from a matrix of thermal features of the first thermographic portion of the thermographic image I-T resulting from acquisitions of thermographic images IT occurred at acquisition time instants included within the time interval when performing the physical activity.

In greater detail, such a matrix of thermal features is obtained in real time, processing the thermographic image acquired at the current instant of time of acquisition and the thermographic images acquired at each previous instant of time of acquisition up to a set number NR of rearward instants of time of acquisition.

For example, if the instants of time are timed per second and NR is equal to 5, the processing at the current instant of time of acquisition takes into account the thermographic images acquired in the last 5 seconds, in which a thermographic image (frame) was acquired every second.

It is thus possible to estimate values of metabolic parameters dependent on the time variable, such as the value of the heart rate or the value of the respiratory rate.

In greater detail, if the estimation algorithm based on the trained regression model involves the use of a neural network, the at least one data processing unit 3 analyses multiple frames within a predetermined time slot (for example 5-10 seconds) by means of a neural network with a structure similar to that described above, also with reference to Figure 4, but which does not consider a single acquired thermographic image (single acquired frame) but a plurality of thermographic images acquired in sequence in a time interval from the thermographic image acquired at the current instant of time considering the images acquired for a plurality of previous instants of time.

Therefore, the estimation algorithm based on the trained regression model considers the variation over time of each thermal feature of the sequence of frames of the time interval to estimate (predict) a value of the heart rate and/or a value of the respiratory rate.

In greater detail, the estimation algorithm is capable of estimating a value of oxygen volume ($VO_2$) that the user U is consuming in an instant of time of acquisition of the at least one thermographic image I-T for each instant of time of acquisition included in the time interval when performing the physical activity on the exercise machine 1.

Such a plurality of estimated values of oxygen volume ($VO_2$) represent a sequence of values from which the data processing unit 3 is capable of determining a temporal variation of the value of oxygen volume ($VO_2$) that the user U is consuming in the time interval when performing the physical activity on the exercise machine 1.

From such a time variation, the data processing unit 3 is capable of determining, in turn, since they are correlated thereto, the value of the heart rate of the user U when performing physical activity on the exercise machine 1 and the value of the respiratory rate of the user U when performing physical activity on the exercise machine 1, as mentioned above.

According to an embodiment, in combination with any of those described above, in order to control the exercise machine 1 when performing physical activity by the user U, the exercise machine 1 is configured to vary the operating parameters of the exercise machine 1 when performing physical activity by the user U based on the estimated metabolic parameters.

"Operating parameters" means speed, inclination and so on, if the exercise machine 1 is a treadmill.

Generally, given a target value of a metabolic parameter based on the goal of the user U, (for example, a target value of $VO_2$ or a target value of RQ), a respective control algorithm of the exercise machine 1 is executed to vary the operating parameters of the exercise machine 1 so as to maintain or bring the value of the metabolic parameter of the user U (for example, the value of RQ) within a range of values centered on the respective target value of the metabolic parameter.

For example, a user U may have assigned as a goal that of maximizing fat consumption.

Fat consumption is related to the intensity of the physical exercise performed by the user U, which can be assessed by means of the volume of oxygen $VO_2$ consumed by the user U and the value of respiratory quotient RQ.

Scientific literature shows a correlation between the percentage value of the oxygen volume $VO_2$ consumed by the user with respect to the maximum value of the oxygen volume $VO_{2max}$ that the user can consume to perform physical activity while maximizing fat consumption (for example, fat consumption is maximum at approximately 38% of $VO_{2max}$).

Having obtained the estimated value of oxygen volume $VO_2$ that the user U is consuming at an instant of time of acquisition of the at least one thermographic image I-T (by using the thermographic camera 2), a data processing module of the exercise machine 1, by means of the implementation of a respective control algorithm, is adapted to modulate (vary) the operating parameters of the exercise machine 1 so that the estimated value of oxygen volume $VO_2$ that the user is consuming remains around that predetermined percentage value for maximizing fat consumption when performing physical activity.

In greater detail, the data processing module of the exercise machine 1 is configured to perform, for example, a tracking or feedback control technique in which the estimated value of the oxygen volume VO2 that the user is consuming is compared with a first reference value and a second reference value representing the endpoints of a range of reference values the central value of which is the reference percentage value (set-point) predetermined for maximizing fat consumption when performing physical activity.

It the estimated value of oxygen volume $VO_2$ that the user is consum ing is between the first reference value and the second reference value, the data processing module of the exercise machine 1 is configured to keep the values of said operating parameters of the exercise machine 1 substantially unchanged so that, from subsequent comparisons, the estimated value of oxygen volume $VO_2$ that the user is consuming is always between the first reference value and the second reference value of the exercise machine 1.

If the estimated value of oxygen volume $VO_2$ that the user is consum ing is lower than the first reference value or higher than the second reference value, the data processing module of the exercise machine 1 is configured to vary the values of said operating parameters of the exercise machine 1 so that, from subsequent comparisons, the estimated value of oxygen volume $VO_2$ that the user is consuming returns to being within the range of reference values delimited by the first reference value and the second reference value.

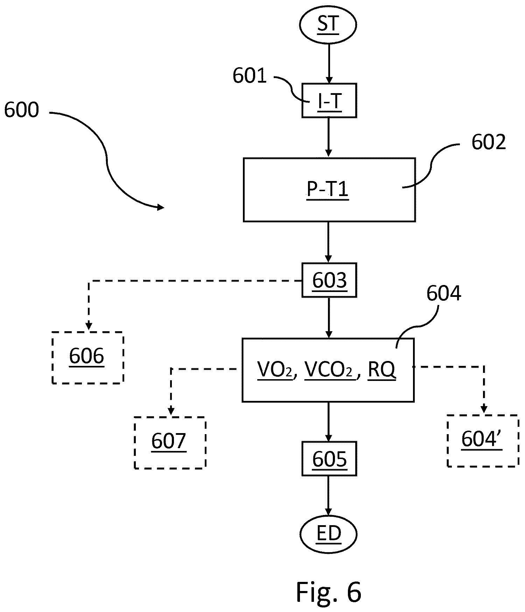

Referring now to Figure 6, a method for determining (estimating or predicting) values of metabolic parameters of a user when performing physical activity is now described, hereinafter also determining method or simply method, according to the present invention.

It should be noted that the components, data and information mentioned below with the description of the method have already been described above with reference to the system 100 and will therefore not be repeated for brevity.

The method 600 comprises a symbolic step of starting ST.

The method 600 comprises a step of acquiring 601, by a thermographic camera 2, at least one thermographic image I-T of at least one portion P-U of the body of the user U when performing physical activity on the exercise machine 1.

The definitions of thermographic camera and thermographic image have been indicated above.

The at least one portion P-U of the body of the user U comprises a body surface P-V of the user U.

The method 600 further comprises a step of identifying 602, by at least one data processing unit 3 operatively connected to said thermographic camera 2, within the at least one thermographic image I-T acquired by the thermographic camera 2, a first thermographic portion P-T1 representative of the body surface P-V of the user U.

As mentioned above, the identification of such a thermographic portion is obtained by the at least one data processing unit 3, for example, by implementing a segmentation technique of the at least one acquired thermographic image I-T.

The method 600 comprises a step of determining 603, by at least one data processing unit 3, for said identified first thermographic portion P-T1, at least one group of thermal features representative of the identified first thermographic portion P-T1.

As mentioned above, "at least one group of thermal features" means one or more thermal features representative of the first thermographic portion P-T1 identified by processing digital pixels of said identified first thermographic portion P-T1.

In greater detail, the at least one data processing unit 3, for said identified first thermographic portion P-T1, determines the at least one group of thermal features representative of the first thermographic portion P-T1 identified by processing digital pixels of said identified thermographic portion P-T1.

The method 600 further comprises a step of estimating 604, by the at least one data processing unit 3, by executing a respective estimation algorithm based on a trained regression model, one or more values of metabolic parameters of the user U when performing physical activity on the exercise machine 1 based on at least one determined group of thermal features representative of said identified first thermographic portion P-T1.

Said one or more values of metabolic parameters of the user U comprise at least one of the following:

- value of oxygen volume ($VO_2$) that the user U is consuming when performing physical activity;
- value of carbon dioxide volume ($VCO_2$) that the user U is producing when performing physical activity by the user;
- value of respiratory quotient or RQ of the user U when performing physical activity by the user.

The method 600 further comprises a step of providing 605, by the at least one data processing unit 3, said one or more values of estimated (or predicted) metabolic parameters.

The method 600 comprises a symbolic step of ending ED.

In accordance with an embodiment, the step of estimating 604, by the at least one data processing unit 3, by executing a respective estimation algorithm based on a trained regression model, one or more values of metabolic parameters of the user U when performing physical activity on the exercise machine 1 is performed by the at least one data processing unit 3 to estimate (or predict) a first value of a first metabolic parameter and a second value of a second metabolic parameter of said one or more values of metabolic parameters.

In this embodiment, shown with dotted lines in Figure 6, the method 600 further comprises a step of determining 604', by the at least one data processing unit 3, a third value of a third metabolic parameter of said one or more values of metabolic parameters based on the estimated first value of the first metabolic parameter and the estimated second value of the second metabolic parameter of said one or more values of metabolic parameters.

In fact, as mentioned above, the value of respiratory quotient or RQ is the ratio between the value of carbon dioxide volume ($VCO_2$) that the user U is producing when performing physical activity and the value of oxygen volume ($VO_2$) that the user U is consuming when performing physical activity.

The value of respiratory quotient RQ provides a representative indication of the intensity of the physical exercise and also allows determining the energy substrates, i.e., how much the user U, during the physical activity, is consuming in terms of fats and sugars.

Conventional values of the respiratory quotient RQ are 0.6 - 0.8 at rest and 1.2 - 1.3 under maximal effort.

Therefore, when considering the relationship RQ = $VCO_2$ / $VO_2$, the data processing unit 3, once estimated two values of metabolic parameters between RQ, $VCO_2$ and $VO_2$, determines the other (not estimated) value of metabolic parameter as a value deriving from the two estimated values of metabolic parameters, using the aforesaid relationship.

In the light of the above, the following cases are envisaged:

a) estimate of values of $VCO_2$ and $VO_2$ -> derived value of RQ = $VCO_2$ / $VO_2$;
b) estimation of values of RQ and $VO_2$ -> derived value of $VCO_2$ = RQ x $VO_2$;
c) estimation of values of RQ and $VCO_2$ -> derived value of $VO_2$ = $VCO_2$ / RQ.

Therefore, the fact of estimating two values of metabolic parameters with the trained regression model and a third value of metabolic parameter only by derivation from the other two estimated values of metabolic parameters represents an apparent advantage also from a computational point of view.

According to an embodiment, in combination with any of the preceding ones, said one or more values of metabolic parameters of the user U further comprise values deriving from one or more of said one or more values of metabolic parameters.

For example, such values deriving from one or more of said one or more values of metabolic parameters comprise:

- value of caloric consumption (kcal/h), correlated to the value of oxygen volume ($VO_2$) that the user is consuming when performing physical activity;
- value of quantity of calories deriving from the consumption of fats and sugars, correlated to the value of respiratory quotient (RQ) of the user U when performing physical activity.

According to an embodiment, in combination with any of the preceding ones, the estimation algorithm based on a trained regression model is based on the use of a regression technique for estimating (or predicting) said one or more values of metabolic parameters of the user U when performing physical activity on the exercise machine 1.

The at least one data processing unit 3 obtains the estimated (or predicted) value of a metabolic parameter of the user U at the instant of time of acquisition of the at least one thermographic image I-T by solving equations of a trained regression model, inserting as input the values of said at least one determined group of thermal features representative of said identified first thermographic portion P-T1.

Examples of linear regression techniques have been described above.

According to an embodiment, as an alternative to the preceding one and in combination with any of the other preceding ones, the estimation algorithm, based on a trained regression model, is based on the execution of a neural network (therefore, by means of the execution of artificial intelligence techniques) for estimating (or predicting) said one or more metabolic parameters of the user U when performing physical activity on the exercise machine 1.

An example of neural network and an example of training of the neural network have been described above.

In accordance with an embodiment, in combination with any of the preceding ones, the step of estimating (or predicting) 604, by executing the estimation algorithm, said one or more values of metabolic parameters of the user U when performing physical activity on the exercise machine 1 is performed by the at least one data processing unit 3 based on, in addition to the determined at least one group of determined thermal features representative of said identified first thermographic portion P-T1, first data D-E representative of the physical exercise being performed by the user U provided by the exercise machine 1 and/or second anthropometric data D-U of the user U.

It is thus possible to obtain a better estimate.

A definition and examples of said first data D-E representative of the physical exercise being performed by the user U provided by the exercise machine 1 have been provided above.

In accordance with an embodiment, in combination with any of the preceding ones and shown in dotted lines in Figure 6, the method 600 further comprises a step of storing 606, by the at least one data processing unit 3, inside at least one memory unit 4 operatively connected to said at least one data processing unit 3, said determined groups of thermal features representative of said identified first thermographic portion P-T1.

In accordance with an embodiment, in combination with any of the preceding ones and shown in dotted lines in Figure 6, the method 600 further comprises a step of storing 607, by the at least one data processing unit 3, inside the at least one memory unit 4 operatively connected to said at least one data processing unit 3, said estimated one or more values of metabolic parameters associated with the acquired at least one thermographic image I-T.

In accordance with an embodiment, in combination with any of those described above, the preceding steps are performed by the at least one data processing unit 3 in an instant of time of acquisition of a single thermographic image I-T acquired by the thermographic camera 2 and based on the processing of said single thermographic image I-T acquired by the thermographic camera 2.

For example, as indicated above, in the case of estimating the value of oxygen volume that the user U is consuming at the instant of time of acquisition of the at least one thermographic image I-T, such a value of metabolic parameter can be estimated from a vector of thermal features (i.e., a single-dimensional matrix), therefore without taking into account the time variable.

In this embodiment, said one or more values of metabolic parameters which can be estimated comprise:

- value of oxygen volume ($VO_2$) that the user U is producing at the instant of time of acquisition of the at least one thermographic image I-T;
- value of carbon dioxide volume ($VCO_2$) that the user U is producing at the instant of time of acquisition of the at least one thermographic image I-T;
- value of respiratory quotient (RQ) of the user U in the instant of time of acquisition of at least one thermographic image I-T.

According to one embodiment, in combination with the preceding one, the at least one data processing unit 3, by executing a respective estimation algorithm based on a trained regression model, estimates (or predicts) a first value of a first metabolic parameter and a second value of a second metabolic parameter of said one or more values of metabolic parameters.

In this embodiment, the at least one data processing unit 3 determines a third value of a third metabolic parameter of said one or more values of metabolic parameters based on the estimated first value of the first metabolic parameter and the estimated second value of the second metabolic parameter of said one or more values of metabolic parameters.

In fact, the value of respiratory quotient or RQ is the ratio between the value of carbon dioxide volume ($VCO_2$) that the user is producing at the instant of time of acquisition of the at least one thermographic image I-T and the value of oxygen volume ($VO_2$) that the user U is consuming at the instant of time of acquisition of the at least one thermographic image I-T.

Therefore, when considering the relationship $RQ = VCO_2 / VO_2$ (already indicated above), the data processing unit 3, once estimated two values of metabolic parameters between RQ, $VCO_2$ and $VO_2$, is configured to estimate the other (not estimated) value of metabolic parameter as a value deriving from the two estimated values of metabolic parameters, using the aforesaid relationship (cases of this type have already been indicated above).

Therefore, also in this embodiment, the fact of estimating two values of metabolic parameters with the trained regression model and a third value of metabolic parameter only by derivation from the other two estimated values of metabolic parameters represents an apparent advantage also from a computational point of view.

According to an embodiment, in combination with any of the preceding ones, wherein the previous steps are performed by the at least one data processing unit 3 in an instant of time of acquisition of a single thermographic image I-T acquired by the thermographic camera 2 and based on the processing of said single thermographic image I-T acquired by the thermographic camera 2, said one or more values of metabolic parameters of the user U further comprise values deriving from one or more of said one or more values of metabolic parameters.

For example, such values deriving from one or more of said one or more values of metabolic parameters comprise:

- value of caloric consumption (kcal/h), correlated to the value of oxygen volume ($VO_2$) that the user is consuming at the instant of time of acquisition of the at least one thermographic image I-T;
- value of the quantity of calories deriving from the consumption of fats and sugars, correlated to the value of respiratory quotient (RQ) of the user U at the instant of time of acquisition of the at least one thermographic image I-T.

In accordance with a further embodiment, in combination with any of the preceding ones, the preceding steps are repeated by the at least one data processing unit 3 for a plurality of instants of time of acquisition included in a time interval and based on the processing of a plurality of thermographic images I-T acquired by the thermographic camera 2 in said time interval.

A thermographic image I-T of said plurality is acquired in an instant of time of acquisition of said plurality of instants of time of acquisition.

In this embodiment, in addition to the values of metabolic parameters already listed above, the estimate of which would thus be more reliable, said one or more values of metabolic parameters of the user U further comprise:

- value of heart rate of the user U when performing physical activity on the exercise machine 1;
- value of respiratory rate of the user U when performing physical activity on the exercise machine 1.

The value of the heart rate or the value of the respiratory rate are in fact correlated to the variation of the value of oxygen volume ($VO_2$) that the user U consumes in a time interval when performing physical activity on the exercise machine 1.

For example, in the case of the estimate of the value of the heart rate of the user in the time interval when performing physical activity by the user on the exercise machine 1, such a value of metabolic parameter can be estimated from a matrix of thermal features of the first thermographic portion of the thermographic image I-T resulting from acquisitions of thermographic images IT occurred at acquisition time instants included within the time interval when performing the physical activity.

In greater detail, such a matrix of thermal features is obtained in real time, processing the thermographic image acquired at the current instant of time of acquisition and the thermographic images acquired at each previous instant of time of acquisition up to a set number NR of rearward instants of time of acquisition.

For example, if the instants of time are timed per second and NR is equal to 5, the processing at the current instant of time of acquisition takes into account the thermographic images acquired in the last 5 seconds, in which a thermographic image (frame) was acquired every second.

It is thus possible to estimate values of metabolic parameters dependent on the time variable, such as the value of the heart rate or the value of the respiratory rate.

In greater detail, if the estimation algorithm based on the trained regression model involves the use of a neural network, the at least one data processing unit 3 analyses multiple frames within a predetermined time slot (for example 5-10 seconds) by means of a neural network with a structure similar to that described above, also with reference to Figure 4, but which does not consider a single acquired thermographic image (single acquired frame) but a plurality of thermographic images acquired in sequence in a time interval from the thermographic image acquired at the current instant of time considering the images acquired for a plurality of previous instants of time.

Therefore, the estimation algorithm based on the trained regression model considers the variation over time of each thermal feature of the sequence of frames of the time interval to estimate (predict) a value of the heart rate and/or a value of the respiratory rate.

In greater detail, the estimation algorithm is capable of estimating a value of oxygen volume ($VO_2$) that the user U is consuming in an instant of time of acquisition of the at least one thermographic image (I-T) for each instant of time of acquisition included in the time interval when performing the physical activity on the exercise machine 1.

Such a plurality of estimated values of oxygen volume ($VO_2$) represent a sequence of values from which the data processing unit 3 is capable of determining a temporal variation of the value of oxygen volume ($VO_2$) that the user U is consuming in the time interval when performing the physical activity on the exercise machine 1.

From such a time variation, the data processing unit 3 is capable of determining, in turn, since they are correlated thereto, the value of the heart rate of the user U when performing physical activity on the exercise machine 1 and the value of the respiratory rate of the user U when performing physical activity on the exercise machine 1.

In accordance with an embodiment, in combination with any of those described above, said trained regression model is obtained following a training step.

Examples of training techniques have been described above.

According to an embodiment, in combination with any of the preceding ones, the body surface P-V of the user U comprised in the at least one portion of the body of the user of the at least one thermographic image I-T acquired is free of clothing (for example the face and neck of the user).

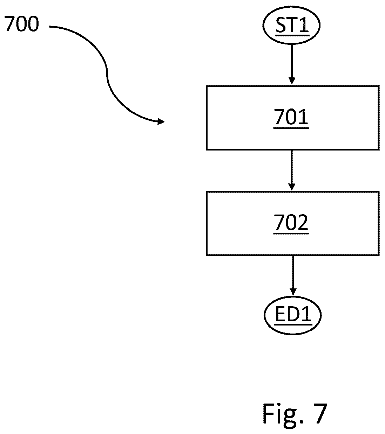

With reference to Figure 7, the present invention also relates to a method 700 for controlling an exercise machine 1 when performing physical activity by the user U.

The method 700 comprises a symbolic step of starting ST1.

The method 700 comprises a step of comparing 701, by a data processing module of the exercise machine 1, one or more values of metabolic parameters estimated by performing the method 600 for determining metabolic parameters of a user U when performing physical activity on an exercise machine 1 described above in accordance with different embodiments, with respective reference values of metabolic parameters for the user U.

The method 700 further comprises a step of checking 702, by the data processing module of the exercise machine 1, at least one operating parameter of the exercise machine 1 based on the outcome of such a comparison (tracking or feedback control).

The method 700 comprises a symbolic step of ending ED1.

The definition of "operating parameters" has been provided above.

Generally, as already mentioned above, given a target value of a metabolic parameter based on the goal of the user U, (for example, a target value of $VO_2$ or a target value of RQ), a respective control algorithm of the exercise machine 1 is executed by the processing module of the exercise machine 1 to vary the operating parameters of the exercise machine 1 so as to maintain or bring the value of the metabolic parameter of the user U (for example, the value of RQ) within a range of values centered on the respective target value of the metabolic parameter.

For example, a user U may have assigned as a goal that of maximizing fat consumption.

Fat consumption is related to the intensity of the physical exercise performed by the user U, which can be assessed by means of the volume of oxygen $VO_2$ consumed by the user U and the value of respiratory quotient RQ.

Scientific literature shows a correlation between the percentage value of the oxygen volume $VO_2$ consumed by the user with respect to the maximum value of the oxygen volume $VO_2$ that the user can consume to perform physical activity while maximizing fat consumption (for example, fat consumption is maximum at approximately 38% of $VO_{2max}$).

Having obtained the estimated value of oxygen volume $VO_2$ that the user U is consuming at an instant of time of acquisition of the at least one thermographic image I-T (by using the thermographic camera 2), the data processing module of the exercise machine 1, by means of the implementation of a respective control algorithm, modulates (varies) the operating parameters of the exercise machine 1 so that the estimated value of oxygen volume $VO_2$ that the user is consuming remains around that predetermined percentage value for maximizing fat consumption when performing physical activity.

In greater detail, the data processing module of the exercise machine 1 performs, for example, a tracking or feedback control technique in which the estimated value of the oxygen volume $VO_2$ that the user is consuming is compared with a first reference value and a second reference value representing the endpoints of a range of reference values the central value of which is the reference percentage value (set-point) predetermined for maximizing fat consumption when performing physical activity.

If the estimated value of oxygen volume $VO_2$ that the user is consuming is between the first reference value and the second reference value, the data processing module of the exercise machine 1 keeps the values of said operating parameters of the exercise machine 1 substantially unchanged so that, also from subsequent comparisons, the estimated value of oxygen volume $VO_2$ that the user is consuming is always between the first reference value and the second reference value of the exercise machine 1.

If the estimated value of oxygen volume $VO_2$ that the user is consuming is lower than the first reference value or higher than the second reference value, the data processing module of the exercise machine 1 varies the values of said operating parameters of the exercise machine 1 so that, from subsequent comparisons, the estimated value of oxygen volume $VO_2$ that the user is consuming returns to being within the range of reference values delimited by the first reference value and the second reference value.

Referring now to Figures 1, 3a, 3b, 3c and 3d, an example of implementation of the method for determining (estimating) metabolic parameters of a user when performing physical activity in accordance with the present invention is described.

A user U steps on an exercise machine 1 (treadmill) and begins the physical activity (running).

A thermographic camera 2, with which the exercise machine 1 is provided, acquires at least one thermographic image I-T of at least one portion P-U of the body of the user U when performing physical activity by the user (Figures 3a and 3b).

The at least one portion P-U of the body of the user U comprises a body surface P-V of the user without clothing (for example, face and neck).

At least one data processing unit 3, operatively connected to said thermographic camera 2, identifies (using segmentation techniques) within the at least one thermographic image I-T acquired by the thermographic camera 2 a first thermographic portion P-T1 representative of the body surface P-V of the user without clothing (for example, face and neck) (segmented thermographic image I-TS in Figure 3c).

The at least one data processing unit 3 performs a subsequent filtering processing of the at least one thermographic image I-T acquired by the thermographic camera 2 using the segmented thermographic image I-TS to obtain a filtered thermographic image I-F (Figure 3d).

The at least one data processing unit 3, for said identified first thermographic portion P-T1, determines the at least one group of thermal features representative of the first thermographic portion P-T1 identified by processing digital pixels of said identified first thermographic portion P-T1.

The at least one data processing unit 3, by executing a respective estimation algorithm based on a trained regression model, estimates one or more values of metabolic parameters of the user U when performing physical activity on the exercise machine 1 based on said at least one determined group of thermal features representative of said identified first thermographic portion P-T1.

Said one or more values of metabolic parameters of the user U comprise at least one of the following: oxygen volume $VO_2$ that the user U is consuming at the instant of time of acquisition of the at least one thermographic image I-T and value of carbon dioxide volume $VCO_2$ that the user U is producing at the instant of time of acquisition of the at least one thermographic image I-T.

The at least one data processing unit 3 provides said one or more values of metabolic parameters, estimated using the trained regression model, to the user and/or to a personal trainer and/or to the exercise machine 1.

A data processing module of the exercise machine 1 controls (varies) at least one operating parameter of the exercise machine 1 when performing physical activity by the user U (for example, speed and/or inclination of the treadmill) based on the outcome of the comparison, by the data processing module of the exercise machine 1, of estimated one or more values of metabolic parameters with respective values of reference metabolic parameters for the user U (tracking).

As it can be noticed, the object of the invention is fully achieved.

In fact, the method and the related system allow estimating metabolic parameters of a user when performing physical activity on an exercise machine in a precise and reliable manner.

Moreover, the method and the related system, not including sensors to be worn by the user, are certainly non-invasive for the user, who is allowed to train in the most optimal conditions possible both in terms of safety and in terms of benefit.

Moreover, the method and the related system appear to be quite simple to implement on an existing fleet of exercise machines, since it is not necessary to adapt an exercise machine for the implementation of the method.

In addition, since no sensors to be worn by the user are included, the method and the related system of the invention are capable of providing metrics related to the intensity of the physical exercise such as to allow the exercise machine to be adjusted so as to maximize the benefits of the training while ensuring compliance with the indications deriving from a prescription or the goal of the user.

Finally, the method and the related system ensure a subsequent control of the exercise machine which avoids having an excessively high intensity of the physical exercise as well as greater safety for the user who does not need, for example, to necessarily wear a heart rate monitor.

In addition, the use of a thermographic camera and the execution of an estimation algorithm based on a trained regression model allows the metabolic intensity of the physical exercise to be estimated more directly with respect to, for example, the use of a "cardio" monitor for detecting the heart rate.

Moreover, the fact that the trained regression model can be loaded into the data processing unit to improve, from a computational point of view, the data processing and the response speed of the data processing unit advantageously allows the system and the related method of the invention to be usable in real-time applications.

Those skilled in the art may make changes and adaptations to the embodiments of the method and system described above or can replace elements with others which are functionally equivalent in order to meet contingent needs without departing from the scope of the following claims. Each of the features described as belonging to a possible embodiment can be achieved irrespective of the other embodiments described.

**Claims**

1. A method (600) for determining values of metabolic parameters of a user (U) when performing physical activity on an exercise machine (1), comprising steps of:

   - acquiring (601), by a thermographic camera (2), at least one thermographic image (I-T) of at least one portion (P-U) of the body of the user (U) when performing physical activity, said at least one portion (P-U) of the body of the user (U) comprising a body surface (P-V) of the user (U);
   - identifying (602), by at least one data processing unit (3) operatively connected to said thermographic camera (2), within the at least one thermographic image (I-T) acquired by the thermographic camera (2), a first thermographic portion (P-T1) representative of the body surface (P-V) of the user (U);
   - for said identified first thermographic portion (P-T1), determining (603), by the at least one data processing unit (3), at least one group of thermal features representative of the identified first thermographic portion (P-T1);
   - estimating (604), by the at least one data processing unit (3), by executing an estimation algorithm based on a trained regression model, one or more values of metabolic parameters of the user (U) when performing physical activity on the exercise machine (1) based on said at least one determined group of thermal features representative of said identified first thermographic portion (P-T1), said one or more values of metabolic parameters of the user U comprising at least one of the following:
   - value of oxygen volume ($VO_2$) that the user (U) is consuming when performing physical activity by the user;
   - value of carbon dioxide volume ($VCO_2$) that the user (U) is producing when performing physical activity by the user;
   - value of respiratory quotient (RQ) of the user (U) when performing physical activity by the user;
   - providing (605), by the at least one data processing unit (3), said estimated one or more values of metabolic parameters.

2. The method (600) according to claim 1, wherein the step of estimating (604), by the at least one data processing unit (3), by running a respective estimation algorithm based on a trained regression model, one or more values of metabolic parameters of the user (U) when performing physical activity on the exercise machine (1) is performed by the at least one data processing unit (3) to estimate a first value of a first metabolic parameter and a second value of a second metabolic parameter of said one or more values of metabolic parameters, the method (600) further comprising a step of determining (604'), by the at least one data processing unit (3), a third value of a third metabolic parameter of said one or more values of metabolic parameters based on the estimated first value of the first metabolic parameter and the estimated second value of the second metabolic parameter of said one or more values of metabolic parameters.

3. The method (600) according to any one of the preceding claims, wherein said one or more values of metabolic parameters of the user (U) further comprise values deriving from one or more of said one or more values of metabolic parameters.

4. The method (600) according to any one of the preceding claims, wherein the estimation algorithm based on a trained regression model is based on the use of a regression technique for estimating said one or more values of metabolic parameters of the user (U) when performing physical activity on the exercise machine (1).

5. The method (600) according to any one of the preceding claims 1 to 3, wherein the estimation algorithm based on a trained regression model is based on the execution of a neural network for estimating said one or more metabolic parameters of the user (U) when performing physical activity on the exercise machine (1).

6. The method (600) according to any one of the preceding claims, wherein the step of estimating (604), by executing the estimation algorithm, said one or more values of metabolic parameters of the user (U) when performing physical activity on the exercise machine (1) is performed by the at least one data processing unit (3) based on, in addition to the determined at least one group of determined thermal features representative of said identified first thermographic portion (P-T1), first data (D-E) representative of the physical exercise being performed by the user (U) provided by the exercise machine (1) and/or second anthropometric data (D-U) of the user (U).

7. The method (600) according to any one of the preceding claims, further comprises a step of storing (606), by the at least one data processing unit (3), inside at least one memory unit (4) operatively connected to said at least one data processing unit (3), said determined groups of thermal features representative of said identified first thermographic portion (P-T1).

8. The method (600) according to any one of the preceding claims, further comprises a step of storing (607), by the at least one data processing unit (3), inside the at least one memory unit ( 4) operatively connected to said at least one data processing unit (3), said estimated one or more values of metabolic parameters associated with the acquired at least one thermographic image (IT).

9. The method (600) according to any one of the preceding claims, wherein the previous steps are performed by the at least one data processing unit (3) in an instant of time of acquisition of a single thermographic image (I-T) acquired by the thermographic camera (2) and based on the processing of said single thermographic image (I-T) acquired by the thermographic camera (2), said one or more values of metabolic parameters which can be estimated comprising:

  - value of oxygen volume ($VO_2$) that the user (U) is producing at the instant of time of acquisition of the at least one thermographic image (I-T);
  - value of carbon dioxide volume ($VCO_2$) that the user (U) is producing at the instant of time of acquisition of the at least one thermographic image (IT);
  - value of respiratory quotient (RQ) of the user (U) in the instant of time of acquisition of at least one thermographic image (I-T).

10. The method (600) according to any one of the preceding claims, wherein the previous steps are repeated by the at least one data processing unit (3) for a plurality of instants of time of acquisition included in a time interval and based on the processing of a plurality of thermographic images (I-T) acquired by the thermographic camera (2) in said time interval, a thermographic image (I-T) of said plurality being acquired in an instant of time of acquisition of said plurality of instants of time of acquisition, said one or more values of metabolic parameters of the user (U) also comprise:

  - value of heart rate of the user (U) when performing physical activity on the exercise machine (1);
  - value of respiratory rate of the user (U) when performing physical activity on the exercise machine (1).

11. The method (600) according to any one of the preceding claims, wherein the body surface (P-V) of the user (U) included in the at least one portion (P-U) of the body of the user (U) of the acquired at least one thermographic image (I-T) is without clothing.

12. A method (700) for controlling an exercise machine (1) when performing physical activity by the user (U), comprising steps of:

  - comparing (701), by a data processing module of the exercise machine (1), one or more values of metabolic parameters estimated by carrying out the method (600) according to any one of the preceding claims, with respective reference values of metabolic parameters for the user (U);
  - checking (702), by the data processing module of the exercise machine (1), at least one operating parameter of the exercise machine (1) based on the outcome of such a comparison.

13. A system (100) for determining metabolic parameters of a user (U) when performing physical activity on an exercise machine (1), comprising:

  - an exercise machine (1) on which a user (U) can perform physical activity;
  - a thermographic camera (2) configured to acquire thermographic images (I-T) of at least one portion (P-U) of the body of the user (U) when performing physical activity on the exercise machine (1);
  - at least one data processing unit (3) operatively connected to the thermographic camera (2);
  - at least one memory unit (4) operatively connected to the at least one data processing unit (3),

the at least one data processing unit (3) being configured to carry out steps of the method for estimating metabolic parameters of a user when performing physical activity on an exercise machine according to any one of the preceding claims.

14. The system (100) according to claim 13, wherein the at least one data processing unit (3) and the at least one memory unit (4) are integrated into the exercise machine (1).

15. The system (100) according to claim 13, wherein the at least one data processing unit (3) and the at least one memory unit (4) reside in a remote electronic processor (200) operatively connected to the thermographic camera (2) and the exercise machine (1).

16. The system (100) according to any one of the preceding claims 13 to 15, wherein the thermographic camera (2) comprises a thermal camera or an infrared camera.

17. The system (100) according to any one of the preceding claims 13 to 16, wherein the thermographic camera (2) is installed on or integrated into the exercise machine (1) and is oriented so as to acquire the thermographic images (I-T) of said at least one portion (P-U) of the body of the user (U) when performing physical activity on the exercise machine (1).

18. The system (100) according to any one of the preceding claims 13 to 16, wherein the thermographic camera (2) is separate from the exercise machine (1) and is installed inside the training space in which the exercise machine (1) usable by the user (U) to perform physical activity is located, the thermographic camera (2) being positioned and oriented so as to acquire the thermographic images (I-T) of said at least one portion (P-U) of the body of the user (U) when performing physical activity on the exercise machine (1).

19. The system (100) according to any one of the preceding claims 13 to 16, wherein the thermographic camera (2) is separate from the exercise machine (1) as it is integrated into a portable electronic device of the user present inside the training space in which the exercise machine (1) usable by the user (U) to perform physical activity is located, the thermographic camera (2) being positioned and oriented so as to acquire the thermographic images of said at least one portion (P-U) of the body of the user (U) when performing physical activity on the exercise machine (1).

EP 4 576 111 A1

Fig. 1

EP 4 576 111 A1

**Fig. 2**

Fig. 3a

Fig. 3b

Fig. 3c

EP 4 576 111 A1

Fig. 3d

Fig. 4

Fig. 5

ST → I-T (601) → P-T1 (602) → 603 → VO₂, VCO₂, RQ (604) → 605 → ED

604'

607

606

600

Fig. 6

700

ST1

701

702

ED1

Fig. 7

Europäisches
Patentamt

European
Patent Office

Office européen
des brevets

# EUROPEAN SEARCH REPORT

**Application Number**

EP 24 21 8485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/046044 A1 (TZVIELI ARIE [US] ET AL) 14 February 2019 (2019-02-14) <br> * abstract; figures 6-9, 28, 35a * <br> * paragraph [0013] - paragraph [0015] * <br> * paragraph [0072] - paragraph [0138] * <br> * paragraph [0163] - paragraph [0164] * <br> * paragraph [0243] - paragraph [0251] * <br> * paragraph [0314] - paragraph [0317] * <br> ----- | 1-19 | INV. <br> G16H20/30 <br> A61B5/00 <br> G16H30/40 <br> G16H40/63 <br> G16H50/20 <br> G16H50/30 |
| A | WO 2022/116032 A1 (QUALCOMM INC [US]; RAMP KATHERYN VICTORIA [US] ET AL.) 9 June 2022 (2022-06-09) <br> * abstract; figures 1c,d * <br> * paragraph [0002] - paragraph [0009] * <br> * paragraph [0046] - paragraph [0059] * <br> ----- | 1-19 | |
| | | | TECHNICAL FIELDS SEARCHED (IPC) <br><br> G16H <br> A61B |

The present search report has been drawn up for all claims

| Place of search | Date of completion of the search | Examiner |
|---|---|---|
| Munich | 14 April 2025 | Menschner, Philipp |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

...........................................................................

& : member of the same patent family, corresponding
 document

EPO FORM 1503 03.82 (P04C01)

## ANNEX TO THE EUROPEAN SEARCH REPORT
## ON EUROPEAN PATENT APPLICATION NO.

EP 24 21 8485

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2025

| Patent document cited in search report | Publication date | Patent family member(s) | | Publication date |
|---|---|---|---|---|
| US 2019046044 A1 | 14-02-2019 | NONE | | |
| WO 2022116032 A1 | 09-06-2022 | CN | 116600708 A | 15-08-2023 |
| | | EP | 4255295 A1 | 11-10-2023 |
| | | JP | 7618799 B2 | 21-01-2025 |
| | | JP | 2024504227 A | 31-01-2024 |
| | | KR | 20230113305 A | 28-07-2023 |
| | | WO | 2022116032 A1 | 09-06-2022 |

EPO FORM P0459

For more details about this annex : see Official Journal of the European Patent Office, No. 12/82